# FASCICULE DE BREVET EUROPEEN

(11) **EP 1 167 945 B1**
(45) Date de publication et mention de la délivrance du brevet: **04.10.2006**
(21) Numéro de dépôt: 01401423.7
(22) Date de dépôt: 31.05.2001
(51) Int. Cl.: G01N 1/22, G01N 33/24

(54) **Dispositif de transport pour analyser des constituants hydrocarbones**
Transportgerät zur Analyse von Kohlenwasserstoffkonstituenten
Transport device for analysing hydrocarbon constituents

(30) Priorité: 28.06.2000 FR 0008445
(43) Date de publication de la demande: 02.01.2002
(73) Titulaire: Institut Français du Pétrole, 92852 Rueil-Malmaison Cedex (FR)
(72) Inventeur: Duriez, Gilbert, 92500 Rueil Malmaison (FR); Dewimille, Bernard, 91100 Corbeil Essone (FR)

(56) Documents cités:
- EP-A1- 1 150 112
- GB-A- 1 569 984
- GB-A- 2 260 812
- US-A- 3 615 235
- US-A- 5 090 256
- US-A- 5 566 720
- US-A- 5 691 809
- US-A- 5 954 862
- US-A- 6 050 150

## Description

La présente invention concerne principalement le domaine des analyses et de mesures des constituants gazeux et/ou liquides qui peuvent être contenus dans les fluides de forage. L'invention s'applique avantageusement aux services dits de « mud logging » qui consistent à effectuer des mesures en cours de forage, notamment sur le fluide de forage en retour de circulation.

On connaît, notamment par le document US-5090256, des méthodes d'extraction des constituants gazeux contenus dans les fluides de forage de manière à détecter la présence desdits constituants dans les roches réservoir traversées. Le principe des mesures consiste à prélever en continu un certain volume de fluide de forage pour le « dégazer » dans un appareil adéquat. Les gaz extraits de l'échantillon de fluide sont ensuite transportés vers une cabine de mesures placée à distance de la tête de puits. La ligne de transport est généralement un tube de plusieurs dizaines de mètres. Les gaz transportés sont ensuite analysés par chromatographie dans la cabine.

Dans l'objectif d'augmenter la précision de quantification et d'élargir la possibilité d'effectuer des mesures sur des hydrocarbures, des tests ont montré que les dispositifs conventionnels ne sont pas suffisamment performants, en particulier pour des constituants hydrocarbonés au-dessus de C4.

L'objet de la présente invention est de fournir une méthode d'analyse permettant d'éviter, ou au moins limiter, les phénomènes de rétention, adsorption et absorption, qui rendent l'analyse qualitative erronée et toute quantification difficile, voire impossible et entraînent des phénomènes de retard à l'analyse qui peuvent provoquer des erreurs d'interprétation sur les zones traversées par le forage. De plus, ces phénomènes d'adsorption et d'absorption peuvent être suivis de relargages non quantifiables qui entraînent une difficulté pour corréler les mesures effectuées en fonction de la profondeur du forage.

Ainsi, la présente invention concerne une utilisation selon la revendication 1. Selon l'invention les moyens de transport comportent une ligne tubulaire comprenant un tube intérieur fabriqué à partir d'au moins un des matériaux plastiques, ou de leurs mélanges, de la liste suivante :
- les polymères fluorés tels que PTFE (polytétrafluoréthylène), FEP (copolymère tétrafluoréthène-perfluoroprène), PVDF (polyfluorure de vinylidène), ETFE (copolymère tétrafluoréthylène éthylène), ETFCE (copolymère éthylène trifluorochloréthylène), PCTFE (polychlorotrifluoréthylène), PFA (perfluoroalkoxyalkane),
- les élastomères fluorés tels que le VITON (marque déposée par Dupont de Nemours) (copolymère hexafluoropropylène/fluorure de vinylidène) ou les terpolymères THV d'hexafluoropropylène/ fluorure de vinylidène/ tétrafluoropropylène ou autres tétrafluoroéthylène-hexafluoropropylène-fluorure de vinylidène traité,
- les élastomères de type Ketone polymères tels que PEEK (polyetherether ketone), mais aussi PEKK, PAEK, PEK, le polycétone aliphatique.

Dans une variante préférée, la ligne de transport peut comporter un tube, en THV ou en PTFE, protégé extérieurement par au moins une autre gaine.

Le tube intérieur selon l'invention peut avoir une épaisseur comprise entre 0,1 et 0,5 mm. Ces matières plastiques sont relativement coûteuses, mais performantes même à faible épaisseur.

Le tube intérieur peut avoir un diamètre interne compris entre 3 et 12 mm, et de préférence entre 6 et 10 mm.

La présente invention sera mieux comprise et ses avantages apparaîtront plus clairement à la lecture des essais ci-après, illustrés par les figures ci-annexées, parmi lesquelles :
- les figures 1, 2, 3 et 8 montrent des moyens de tests,
- les figures 4, 5a, 5b, 6a, 6b, 7, 9, 10, 12 et13 montrent les mesures effectuées à l'aide d'un spectrographe.
- La figure 11 illustre un tube utilisé selon l'invention.

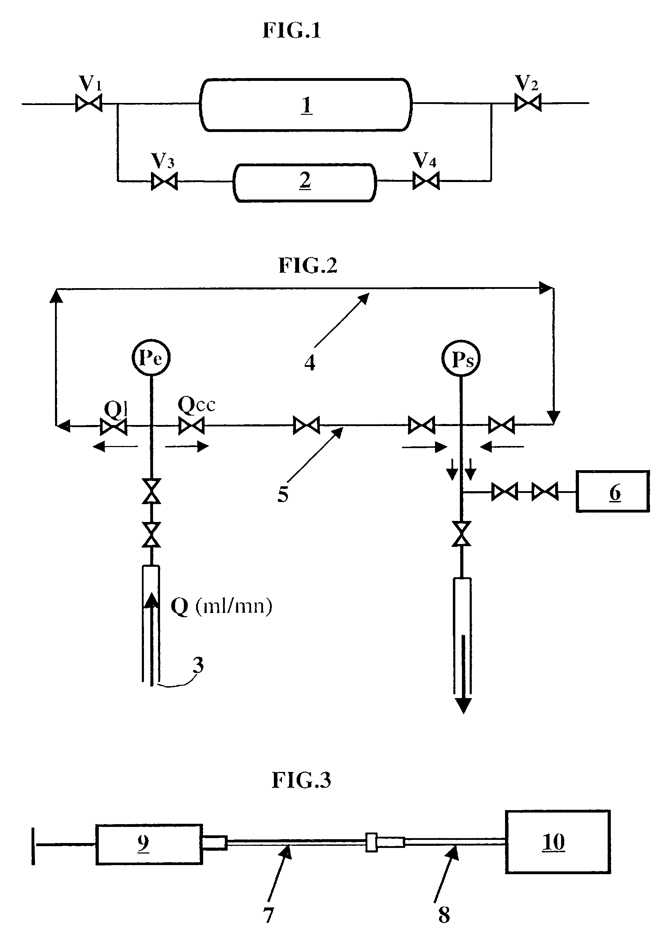

La préparation de l'échantillon injecté se fait à l'aide du système de la figure 1.

Cet appareil est constitué de deux ampoules 1 et 2 en verre que l'on peut isoler chacune par deux vannes V3 et V4. Les vannes V1 et V2 isolent l'ensemble des deux ampoules.

On remplit l'ampoule 2 avec le ou les hydrocarbures liquides. Après avoir fermé les vannes V3 et V4, on fait le vide dans le reste du montage. Puis on sature l'autre partie avec le ou les gaz d'hydrocarbures. On isole l'appareil en fermant V1 et V2. On ouvre V3 et V4 pour mettre en mélange le gaz et le liquide (renforcé par une légère agitation). Après quelques instants et après vidange complète de tout liquide de l'ampoule 1, on ferme V3 et V4. On obtient un mélange d'hydrocarbure léger saturé en lourd (le volume est d'environ 35cc) prêt à être injecté. On peut également faire des mélanges binaires de gaz avec cet appareil. Cet appareil a permis d'obtenir un mélange de méthane saturé en toluène (concentration approximative de 2%).

La figure 2 montre un montage de test en tube inox ¼ qui permet, lors d'une injection d'un échantillon gazeux dans l'entrée 3, de séparer celui-ci en deux parties Ql et Qcc, respectivement dans la ligne 4 à tester, et le court-circuit 5 en tube inox. Différentes vannes et régulateurs complètent le circuit. La référence 6 désigne les moyens d'analyse, par exemple un spectrographe de masse.
- La ligne de court-circuit 5 permet d'analyser plus rapidement l'échantillon dans le spectrographe.
- La ligne 4 est constituée d'une longueur variable de tube en différents matériaux testés.

On admet que la partie « court-circuit » est pratiquement sans perte de constituants, le signal résultant de la mesure du mélange issu de cette branche, apparaissant sur le spectrographe plus tôt que celui résultant du débit Ql passant par la ligne 4, peut servir de référence.

La figure 3 montre un montage simplifié permettant de tester un certain nombre de tubes en différents matériaux. Le tube à tester 7 est fixé sur un tube 8 en PEEK de longueur 1,5 m et de diamètre interne égal à 250 µm. Pour obtenir des conditions de tests comparatifs, on utilise un spectromètre de masse 10 relié au capillaire 8 en PEEK supposé sans pertes. Une seringue 9 permet l'injection de l'échantillon de constituants.

Le mélange injecté se compose de :
- 1 cc de vapeurs prises dans le ciel gazeux d'une bouteille de toluène,
- 9 cc de méthane.

L'injection se fait par aspiration d'une faible partie de ce mélange au niveau de l'entrée d'air du montage.

A l'aide de ce montage, on teste plusieurs tubes d'une longueur de 50 cm en matériaux différents de façon à déterminer celui, ou ceux, qui transporte correctement les composés les plus lourds, tels que le toluène. On a testé un tube en verre, en verre traité, un tube de polyéthylène, un tube « à vide » en caoutchouc, un tube de faible épaisseur en THV. Le verre traité (inerté chimiquement) est baigné dans du diméthyldichlorosilane. Après plusieurs rinçages à l'aide de méthanol anhydre, le tube est séché à une température d'environ 95°C.

Après une injection de C1 pur, on a mesuré les quantités de composés relargués.

De ces essais, sont sorties deux familles de produits :
- une première famille comprend le verre, le verre traité et le THV où l'adsorption est peu importante. Le THV a présenté des résultats paraissant moins intéressants mais la section du tube en THV (ID=7,3 mm, au lieu de 4 mm pour les autres tubes) étant plus importante que celles des autres tubes, atténue la qualité des réponses à cause de la réduction de la vitesse de circulation des effluents.
- Les résultats avec le tube à vide (caoutchouc) et le polyéthylène sont très négatifs. Ces tubes absorbent pratiquement complètement les hydrocarbures sans les relarguer après un lavage au méthane.

Ces essais ont conduit à tester des tubes plastiques en THV, en PTFE, en PEEK présentant tous des propriétés de très faible perméabilité aux hydrocarbures, même à faible épaisseur de l'ordre de quelques dixièmes de millimètre.

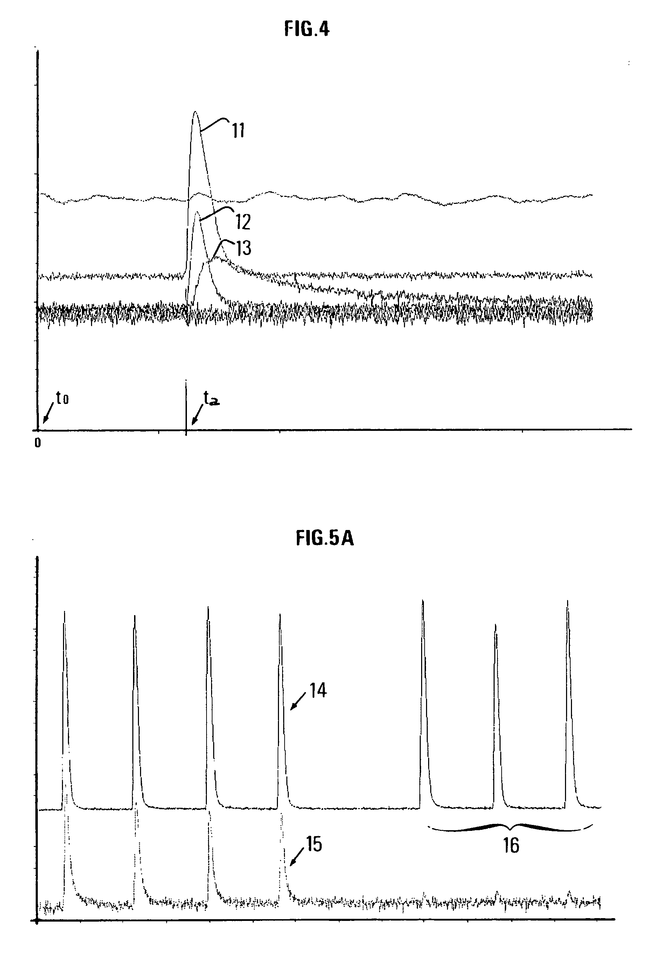

La figure 4 montre l'injection d'un mélange d'hydrocarbures gazeux dans un tube de chlorure de polyvinyle, à l'aide du montage selon la figure 2. Le tube de PVC a 50 m de long et un diamètre de 10 mm. Le mélange injecté en 3 comprend du C1, du C5 et du benzène.

L'injection a lieu au temps t0, son arrivée détectée par le spectrographe au temps ta. Le pic 11 correspond au C1, le pic 12 correspond au C5 et le pic 13 au benzène. La forme homothétique de pics 11 et 12 montre que le transport des C1 et C5 est régulier et sensiblement complet. Par contre, la forme traînante du signal 13 du benzène montre qu'il y un mauvais transport.

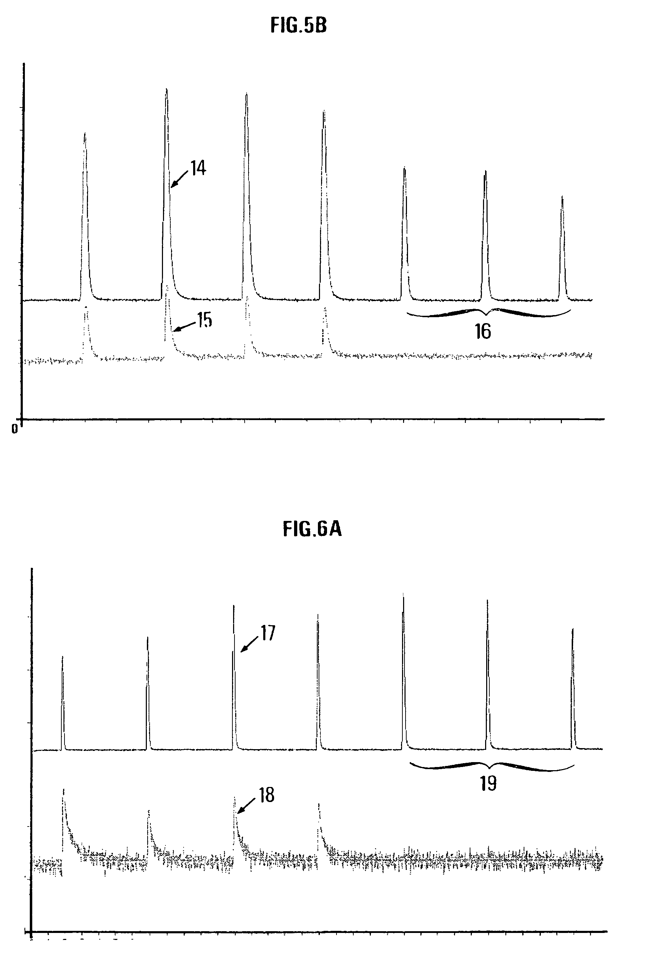

Les figures 5a et 5b montrent les résultats de tests effectués à l'aide du montage selon la figure 2 avec un tube en THV de 50 m de long et de 7,3 mm de diamètre intérieur, épaisseur inférieure à 0,5 mm. Le mélange injecté comprend du C1 et du C7 en concentration correspondante à un rapport molaire C7/C1 égal à 0,4%.

Les conditions de l'essai de la figure 5a sont les suivantes Q=93 ml/min, Ps=20 mb, Pe=34 mb. L'essai de la figure 5b diffère du précédent par un débit d'injection de 21 ml/min. Ces deux essais permettent de comparer les résultats pour des temps de transit différents.

Les pics 14 et 15 représentent respectivement les réponses à l'arrivée du C1 et du C7. La forme comparable des pics, ainsi que leur régularité, montre que le transport dans un tube en THV est excellent. Les pics de C1 des zones 16 des figures 5a et 5b résultent d'un lavage de la ligne en THV par du C1 pur. L'absence de pics de C7 dans ces mêmes zones montrent qu'il n'y pas de C7 adsorbé dans la ligne de THV.

De la même façon que pour le tube en THV, on teste un tube de polyéthylène de longueur 100 m et de diamètre intérieur 10 mm, extérieur 12 mm, dans les mêmes moyens et à partir d'un échantillon injecté identique.

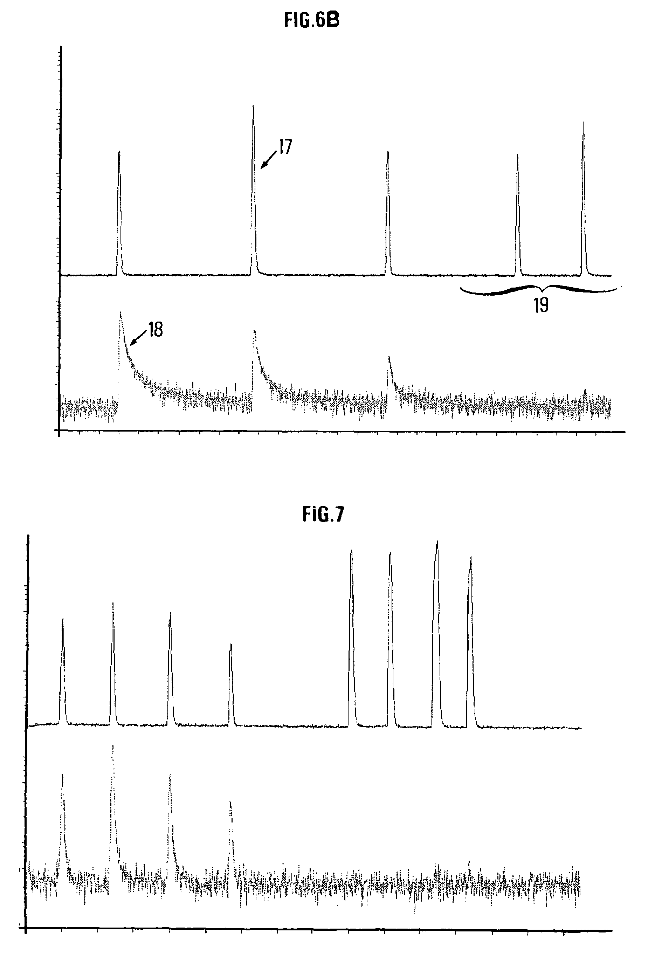

La figure 6a correspond à un débit de 93 ml/min, la figure 6b à un débit de 50 ml/min.

Les pics 17 et 18 correspondent respectivement au C1 et C7. Les zones 19 représentent le lavage au C1 pur.

Le signal 18 relatif au toluène est beaucoup plus large et traînant dans le tube polyéthylène. Cette dégradation s'accentue avec la diminution de débit, c'est à dire avec une augmentation du temps de transit.

En comparaison avec le tube THV, le tube en polyéthylène est nettement moins performant pour le transport des hydrocarbures lourds, même si l'adsorption du toluène ne semble pas irréductible, il est clair qu'il a des perturbations dans le déplacement du toluène dans un tube en polyéthylène. De plus, la variation du temps de transit influe sur la qualité du signal reçu lors du transport dans le polyéthylène contrairement au transport dans le tube en THV.

La figure 7 montre les résultats obtenus dans des conditions de basse température (2°C). La ligne de THV selon le montage de la figure 2 est placée dans un système réfrigérant. Les résultats de transport de C1 et C7 visibles sur la figure 7 démontrent qu'ils sont sensiblement identiques à ceux obtenus à température ambiante.

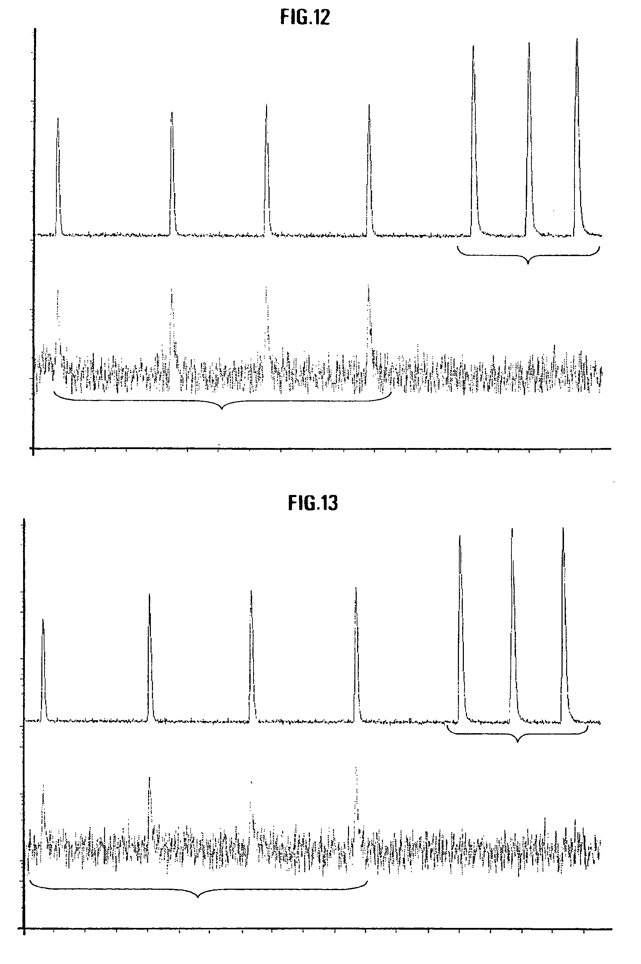

Les figures 12 et 13 montrent les résultats des tests obtenus à partir du montage selon la figure 2 pour comparer les performances du PTFE (figure 12) par rapport au THV (figure 13). Le gaz étalon utilisé ici comprend 10% de C1, 1000 ppm de benzène , 500 ppm de toluène, le reste est de l'azote.

Les conditions d'essais sont: PTFE (figure 12) Q=93 ml/min; Ps=20 mb ; Pe=27 mb. Le diamètre intérieur de la ligne est 8 mm, une épaisseur de 1 mm, et a 50 m de long.

Les conditions d'essais sont: THV (figure 13) Q=93 ml/min ; Ps=20 mb ; Pe=28 mb. Le diamètre intérieur de la ligne est 7,3 mm, une épaisseur inférieure à 0,5 mm et a 50 m de long.

Les mesures sont identiques et montrent donc des performances semblables entre le PTFE et le THV.

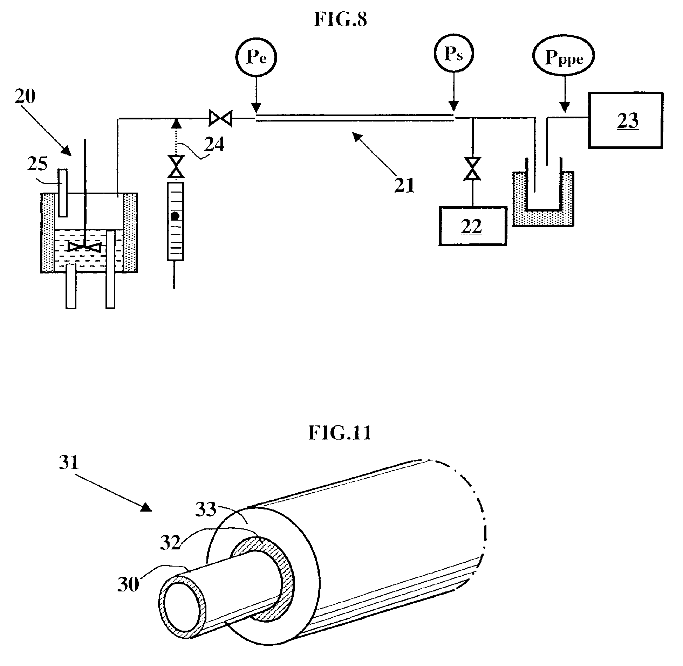

La figure 8 représente le schéma d'un ensemble d'extraction 20, ou dégazeur, une ligne de transport 21 du gaz prélevé, une installation de mesure ou d'analyse 22, une pompe à vide 23 pour faire fonctionner le dégazeur 20 et la ligne 21 en dépression (on notera que toutes les valeurs de pression données sont en pression absolue). Le réglage peut être effectué par une restriction de l'admission d'air 25. La référence 24 désigne des moyens de contrôle intermittent du débit. L'admission d'un échantillon de gaz C7/C1 peut se faire en 24 ou en 25. La ligne 21 est en tube THV de 50 mètres de long. Les conditions de fonctionnement sont Pe=34 mb, Ps=20 mb et Q=93 ml/min. L'échantillon de gaz peut être également injecté par 25 dans l'eau remplissant le carter du dégazeur 20.

On cite en référence le document FR 99/12.032 qui décrit un dégazeur selon la figure 8.

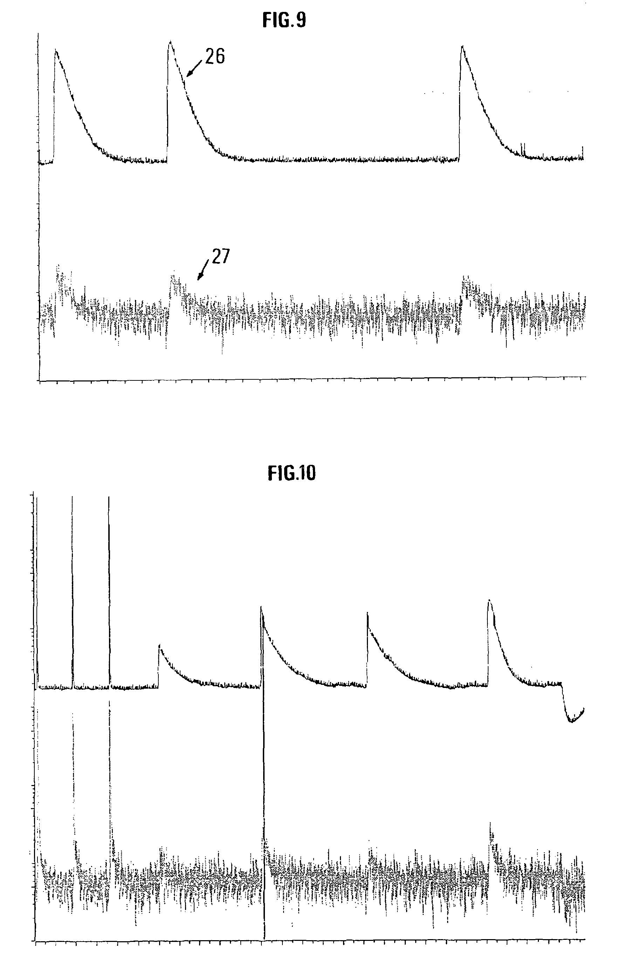

La figure 9 montre les résultats de mesures de C1 (pic 26) et C7 (pic 27). Il apparaît que dans les conditions d'une installation industrielle, le transport de C7 est correctement effectué grâce à une ligne en THV. L'injection de l'échantillon dans le liquide du dégazeur ou en entrée de la ligne ne change pas les résultats.

La figure 10 donne les résultats d'essai effectué à l'aide d'un ensemble selon la figure 8, mais en fonctionnement atmosphérique au niveau du dégazeur. Un tel dégazeur est par exemple décrit dans le document US-5090256, cité ici en référence. L'échantillon de gaz C1 et C7 a été injecté dans de la boue de forage de densité 1,2 placée dans le dégazeur. Les trois premiers pics correspondent à la réponse à trois injections successives de gaz directement à l'entrée de la ligne, à titre de comparaison avec le signal obtenu à la suite de l'injection de l'échantillon dans la boue.

On note que la présence de C7 est reconnue par la mesure dans le spectromètre de masse, donc le transport est correct dans la ligne 21 selon l'invention. Dans des conditions similaires, avec une ligne en polyvinyle comme classiquement utilisée, le C7 n'est pas détecté au spectromètre et le C1 est sous évalué.

La figure 11 montre schématiquement une réalisation d'une ligne selon l'invention où le tube interne 30 est en THV, recouvert d'un ensemble 31 composé par exemple, d'une couche 33 d'élastomère ou de PE comme couche de protection mécanique, armée ou non. Cette couche est de préférence collée sur le tube 30 par une couche de matière adhésive 32. Le tube 30 peut avoir 7,3 mm de diamètre interne, pour une épaisseur inférieure à environ 0,5 mm, et de préférence inférieure à 0,2 mm. Le diamètre extérieur de l'ensemble du tube est d'environ 13 mm.

## Revendications

1. Utilisation due ligne tubulaire pour constituer des moyens de transport entre des moyens d'extraction sous forme gazeuse des hydrocarbures contenus dans un fluide de forage et des moyens d'analyses et de mesures sur ces gaz extraits, **caractérisée en ce qu'**il s'agit d'une ligne tubulaire de plusieurs dizaines de mètres comprenant un tube intérieur fabriqué à partir d'au moins l'un des matériaux plastiques, ou de leurs mélanges, de la liste suivante :
• les polymères fluorés tels que PTFE (polytétrafluoréthylène), FEP (copolymère tétrafluoréthène-perfluoroprène), PVDF (polyfluorure de vinylidène), ETFE (copolymère tétrafluoréthylène éthylène), ETFCE (copolymère éthylène trifluorochloréthylène), PCTFE (polychlorotrifluoréthylène), PFA (perfluoroalkoxyalkane),
• les élastomères fluorés, tels les copolymères hexafluoropropylène/fluorure de vinylidène, les terpolymères THV d'hexafluoropropylène/ fluorure de vinylidène/ tétrafluoropropylène, les tétrafluoroéthylène-hexafluoropropylène-fluorure de vinylidène traité,
• les élastomères de type Ketone polymères, tels que PEEK (polyetherether ketone), PEKK, PAEK, PEK, le polycétone aliphatique permettant d'éviter, ou au moins de limiter, les phénomènes de rétention, adsorption et absorption desdits hydrocarbures, d'un dispositif d'analyse et/ou de mesure en continu en cours d'une opération de forage utilisant ledit fluide.

2. Utilisation selon la revendication 1, dans lequel ledit tube intérieur est en THV.

3. Utilisation selon l'une des revendications précédentes, dans lequel ledit tube intérieur est protégé extérieurement par au moins une autre gaine.

4. Utilisation selon l'une des revendications précédentes, dans lequel le tube intérieur a une épaisseur comprise entre 0,1 et 0,5 mm, et de préférence inférieure à 0,2 mm.

5. Utilisation selon l'une des revendications précédentes, dans lequel le tube intérieur a un diamètre interne compris entre 3 et 12 mm, et de préférence entre 6 et 10 mm.

## Claims

1. Use of a tubular line to form transport means between means for extraction in gaseous form of the hydrocarbons contained in a drilling fluid and means for analysing and measuring these extracted gases, **characterised by** the fact that it is a tubular line of several tens of metres including an internal tube made of at least one of the plastics materials, or of their mixtures, from the following list:
• fluorinated polymers such as PTFE (polytetrafluoroethylene), FEP (tetrafluoroetheneperfluoroprene copolymer), PVDF (polyvinylidene fluoride), ETFE (tetrafluoroethylene ethylene copolymer), ETFCE (ethylene trifluorochloroethylene copolymer) PCTFE (polychlorotrifluoroethylene), PFA (perfluoroalkoxyalkane),
• fluorinated elastomers, such as hexafluoropropylene/vinylidene fluoride copolymers, THV hexafluoropropylene/vinylidene fluoride/tetrafluoropropylene terpolymers, treated tetrafluoroethylene-hexafluoropropylene-vinylidene fluoride,
• elastomers of ketone polymer type, such as PEEK (poly ether ether ketone), PEKK, PAEK, PEK, aliphatic polyketone,
permitting prevention, or at least limitation, of phenomena of retention, adsorption and absorption of the said hydrocarbons, of a device for continuous analysis and/or measurement during a drilling operation using the said fluid.

2. Use as described in claim 1, in which the said internal tube is made of THV.

3. Use as described in one of the preceding claims, in which the said internal tube is protected externally by at least one other sheath.

4. Use as described in one of the preceding claims, in which the internal tube has a thickness of between 0.1 and 0.5 mm, and preferably less than 0.2 mm.

5. Use as described in one of the preceding claims, in which the internal tube has an internal diameter of between 3 and 12 mm, and preferably between 6 and 10 mm.

## Patentansprüche

1. Verwendung eines Rohrstranges um Transportmittel zu bilden zwischen Extraktionsmitteln in gasförmiger Form der Kohlenwasserstoffe, die in einer Bohrflüssigkeit enthalten sind, und Analyse- und Messmittel dieser extrahierten Gase, **dadurch gekennzeichnet, dass** es sich um einen Rohrstrang von mehreren Dutzend Metern handelt, mit einem Innenrohr, das aus zumindest einem der plastischen Materialien aus der nachstehenden Liste, oder aus deren Mischungen, hergestellt ist:
- die Fluorpolymere wie PTFE (Polytetrafluorethylen), FEP (Fluorethylenpropylen), PVDF (Polyvenylidenfluorid), ETFE (Ethylentetrafluorethylen), ETFCE (Ethylen-Trifluorochlorethylen-Copolymer), PCTFE (Polychlortrifluorethylen), PFA (Perfluoralkoxypolymer),
- die Fluorelastomere, wie die Copolymere Hexafluoropropylen/Vinylidenfluorid, die Terpolymere THV von Hexafluoropropylen/Vinylidenfluorid/Tetrafluoropropyle n, die behandelten Tetrafluoroethylen-Hexafluoropropylen-Vinylidenfluoride,
- die Elastomere des Typs Ketonpolymere, wie PEEK (Poly-Ether-Ether-Keton), PEKK, PAEK, PEK, das aliphatische Polyceton, die es erlauben, die Phänomene der Retention, der Adsorption und der Absorption der Kohlenwasserstoffe von einer kontinuierlichen Analyse und/oder Messeinheit während eines Bohrvorgangs, der diese Flüssigkeit verwendet, zu verhindern oder zumindest zu begrenzen.

2. Einheit nach Anspruch 1, in der das Innenrohr aus THV ist.

3. Einheit nach einem der vorhergehenden Ansprüche, in der das Innenrohr außen durch zumindest ein anderes Rohr geschützt ist.

4. Einheit nach einem der vorhergehenden Ansprüche, in der das Innenrohr eine Dicke zwischen 0,1 und 0,5 mm, und vorzugsweise unter 0,2 mm aufweist.

5. Einheit nach einem der vorhergehenden Ansprüche, in der das Innenrohr einen Innendurchmesser zwischen 3 und 12 mm, und vorzugsweise zwischen 6 und 10 mm aufweist.
